# EUROPÄISCHE PATENTSCHRIFT

(11) **EP 0 642 366 B1**
(45) Veröffentlichungstag und Bekanntmachung des Hinweises auf die Patenterteilung: **08.10.1997**
(21) Anmeldenummer: 93909948.7
(22) Anmeldetag: 11.05.1993
(51) Int. Cl.: A61M 15/00

(54) **VORRICHTUNG ZUR ERZEUGUNG INHALIERBARER WIRKSTOFFPARTIKEL**
DEVICE FOR GENERATING INHALABLE ACTIVE SUBSTANCE PARTICLES
DISPOSITIF GENERATEUR DE PARTICULES INHALABLES DE SUBSTANCES ACTIVES

(30) Priorität: 29.05.1992 DE 4217787
(43) Veröffentlichungstag der Anmeldung: 15.03.1995
(73) Patentinhaber: GGU GESELLSCHAFT FÜR GESUNDHEITS- UND UMWELTFORSCHUNG MBH & CO. VERTRIEBS KG, D-65933 Frankfurt (DE)
(72) Erfinder: HEIDE, Helmut, D-6233 Kelkheim 2 (DE); HUGEMANN, Bernhard, D-6000 Frankfurt/M. 70 (DE); PABST, Joachim, D-6107 Reinheim (DE)
(74) Vertreter: Roesner, Werner
(86) Internationale Anmeldenummer: EP9301157
(87) Internationale Veröffentlichungsnummer: WO9324165

(56) Entgegenhaltungen:
- EP-A- 0 407 028
- WO-A-92/04067
- US-A- 4 841 964

## Beschreibung

Die Erfindung betrifft eine Vorrichtung zur mechanischen Erzeugung inhalierbarer, vorzugsweise lungengängiger Wirkstoffpartikel von einem verfestigten Arzneimittelvorrat und zur Führung der Wirkstoffpartikel in den Atemtrakt.

Aus der europäischen Patentanmeldung 0407028 A2 ist eine Vorrichtung bekannt, bei der mittels rotierender Messer von einem verfestigten Arzneimittelvorrat eine bestimmte Dosis von Wirkstoffpartikeln abgetragen wird. Hierzu ist es notwendig, den oberen Teil der Vorrichtung gegen die Messer zu drücken und dabei zu drehen. Die Führung der Wirkstoffpartikel erfolgt durch Ansaugen von Luft am unteren Teil der Vorrichtung. Hierbei werden zwei Luftströme erzeugt, einer durch den oberen Teil hindurch und einer durch Bypassöffnungen im unteren Teil.

Aus der DE-OS 40 27 390 ist eine Vorrichtung bekannt, bei der mittels einer rotierenden Bürste von einem verfestigten Arzneimittelvorrat Wirkstoffpartikel abgebürstet werden. Durch einen vorgespannten Reibradantrieb wird die Bürste in Drehung versetzt. Die abgebürsteten Wirkstoffpartikel werden mittels eines Luftstromes inhaliert, der von unten durch die Vorrichtung angesaugt wird.

Beide Vorrichtungen haben den Nachteil, daß sie in ihrer Dosiergenauigkeit sehr ungenau sind. Ferner kann es durch die Art der Luftführungen bedingt leicht zu einer Agglomeration der Wirkstoffpartikel kommen. Bei der EP 407 028 fallen die abgeschabten Wirkstoffpartikel nach unten auf den Boden des unteren Teiles der Vorrichtung.

Mittels zweier Luftströme soll die Inhalation erfolgen. Es ist evident, daß eine derartige Luftführung leicht außer Kontrolle geraten kann, wenn sich die Strömungswiderstände der beiden Luftwege durch eine Fehlbedienung ändern, z. B. durch unbeabsichtigtes Zuhalten der Lufteintrittsöffnungen oder durch Verstopfen der Öffnungen aufgrund von Wirkstoffbruchstücken. Bei der DE-OS 4027 390 müssen die abgebürsteten Wirkstoffpartikel um die Bürsteneinrichtung herum und durch die Bürste hindurch angesaugt werden. Agglomerationen sind hierdurch unvermeidbar.

Aufgabe der Erfindung ist es, eine Vorrichtung zu schaffen, mit der bei einfachster Bedienungsart von einem verfestigten Arzneimittelvorrat in reproduzierbarer Weise genau dosierbare Wirkstoffpartikelmengen inhaliert werden können.

Die Aufgabe der Erfindung wird durch gelöst die Merkmale des Anspruchs 1.

Auf diese Weise wird ein Strömungspfad geschaffen, bei dem die engste Stelle zwischen den Schneiden der Stirnfräse liegt, gegen die der Wirkstoff-Ringkörper angedrückt wird. Beim Inhalieren einer Luftmenge von 1 l/sec. entstehen Strömungsgeschwindigkeiten bis zu ca. 200 km/h. Die von der Stirnfräse generierten Partikel werden im Zeitpunkt ihrer Entstehung abtransportiert und können mit nachfolgenden Partikeln nicht agglomerieren. Durch diesen Jet-Effekt wird ferner eine deutliche Spreizung der Partikelkonzentration im Luftstrom erreicht. Die Dosierungsmenge wird durch die Anzahl der Umdrehungen der Stirnfräse bestimmt. Die Größe der Partikel wird im wesentlichen durch die Geometrie des Stirnfräsers, durch die Festigkeit des Wirkstoff-Ringkörpers und dem Anpreßdruck des Ringkörpers gegen die Stirnfräse sowie durch die Luftgeschwindigkeit an den Schneiden bestimmt.

Der Wirkstoffkörper ragt aus dem Inhalationsrohr heraus. Damit dieser rechtzeitig vor endgültigem Verbrauch ausgetauscht werden kann, ist das Inhalationsrohr mit einer Hubbegrenzung versehen.

Der Ringkörper ist zusammen mit dem Inhalationsrohr austauschbar angeordnet. Hierzu ist lediglich erforderlich, den Oberteil der Vorrichtung abzunehmen.

Ein sehr wichtiger Gesichtspunkt der Erfindung ist die geometrische Gestaltung des Wirkstoffkörpers. Dessen ringförmige Ausbildung hat folgende Vorteile:

Der innere Hohlraum des Ringkörpers ist Teil des Inhalationsrohres, durch das die Wirkstoffpartikel unmittelbar nach ihrer Generierung mit der Inhalationsluft durchströmen. Tote Strömungszonen und damit Ablagerungen von Partikeln werden hierdurch vermieden. Bei einem Ring kann die Wandstärke so gewählt werden, daß die Unterschiede der Schnittgeschwindigkeiten der Stirnfräse am inneren und äußeren Ringdurchmesser in bezug auf die Gesamtfläche zu vernachlässigen sind. Bekanntlich wäre die Schnittgeschwindigkeit im Zentrum eines Vollkörpers Null. Dies würde zu einem undefinierten Abtrag vom Wirkstoffkörper und damit zu einer undefinierten Partikelgenerierung führen.

Die Vorrichtung kann für medikamentös unterschiedliche Arten von Wirkstoffkörpern verwendet werden. Um Verwechslungen zu vermeiden, weist das Inhalationsrohr eine schlüsselartige Kennung auf, die jeweils auf ein bestimmtes Arzneimittel abgestimmt ist.

Die Vorrichtung kann auch zum Inhalieren durch die Nase verwendet werden.

Die Rotation der Stirnfräse wird durch einen Federantrieb ausgelöst, wobei die Anzahl der Umdrehungen vorbestimmbar ist. Auch die Verwendung von batteriebetriebenen Miniatur-Getriebe-Elektromotoren ist möglich.

Unabhängig hiervon ist die Zeit der Partikelgenerierung deutlich kürzer als die gesamte Inhalationszeit. Sie beträgt im allgemeinen nur Bruchteile einer Sekunde. Die Koordinierung der Partikelgenerierung und des Inhalationsvorganges sind durch die Erfindung damit für den Patienten unproblematisch.

In den Zeichnungen ist die Erfindung näher beschrieben.
- Fig. 1: zeigt im Schnitt die erfindungsgemäße Vorrichtung,
- Fig. 2: eine Draufsicht auf die Stirnfräse und
- Fig. 3: den Strömungspfad der angesaugten Luft in der Vorrichtung.

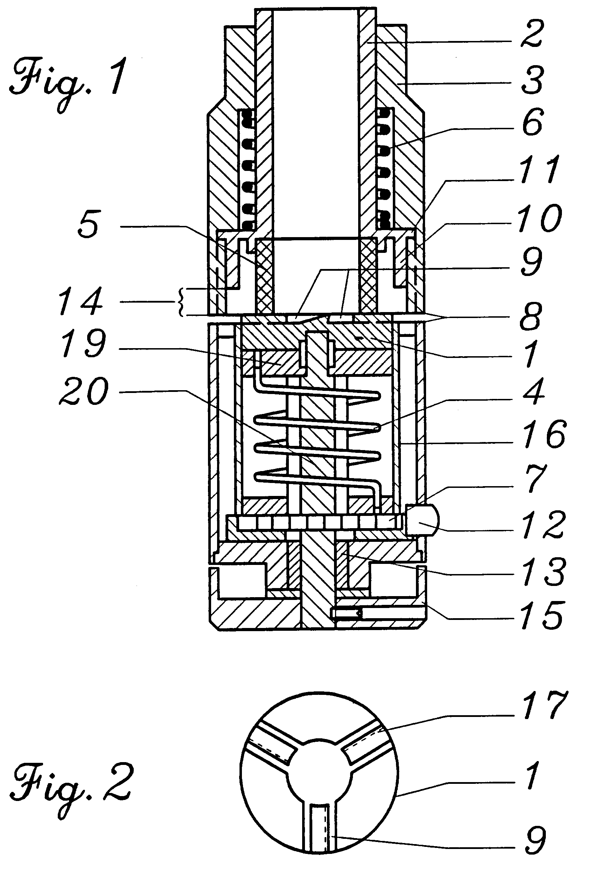

In Fig. 1 ist die Stirnfräse 1 dargestellt, die auf einer Welle 20 befestigt ist. Auf der Welle 20 befindet sich ferner ein Rastrad 7 und am unteren Ende eine Rücklaufsperre 13. Im Bereich des Federmotorgehäuses 16 ist eine Antriebsfeder 4 vorgesehen, welche die Welle 20 konzentrisch umgibt. Die Antriebsfeder 4 ist einerseits im oberen Teil 19 des Federmotorgehäuses 16 befestigt und andererseits im Rastrad 7. Durch Drehen des Aufzugsknopfes 15 wird die Feder 4 gespannt. Durch Drücken des Auslösemechanismus 12 wird die Stirnfräse 1 in Drehung versetzt.

Der obere Teil der Vorrichtung weist das Inhalationsrohr 2 auf, an dessen unterem Ende der Arzneistoffvorrat 5 in Form eines verfestigten Wirkstoff-Ringkörpers eingesetzt ist. Das Inhalationsrohr 2 wird zusammen mit dem Arzneistoffvorrat 5 mittels Andruckfeder 6 gegen die Stirnfräse 1 gedrückt. Die Stirnfläche des Arzneistoffvorrates 5 liegt damit immer auf der Stirnfräse 1 auf. Am unteren Ende des Inhalationsrohres 2 befindet sich ein Vorsprung 10 der als Verbrauchsbegrenzer dient. Wenn dieser auf dem Inhalatorgehäuse aufsitzt, kann keine Generierung mehr erfolgen. Der Verbrauchsbereich ist durch die beiden Pfeile 14 dargestellt. Ferner sind als Kennung für den Wirkstoff und die hierzu eingestellten Parameter der Vorrichtung an dieser Stelle zwei Bolzen 11 angeordnet, die in Verbindung mit dem Mundstück 3 als Schlüsselstecker wirken.

Der obere und der untere Teil der Vorrichtung sind mittels nicht dargestellter Führungszapfen verbunden. Zwischen dem oberen und unteren Teil ist ein Ringspalt 8 vorhanden, der als Lufteinlaß dient. Dieser ist, wie aus Fig. 1 ersichtlich, im Bereich der Schneiden 17 der Stirnfräse 1 angeordnet.

Die Fig. 2 zeigt in Draufsicht die Schneiden 17 und die Vertiefungen 9 vor und hinter den Schneiden 17.

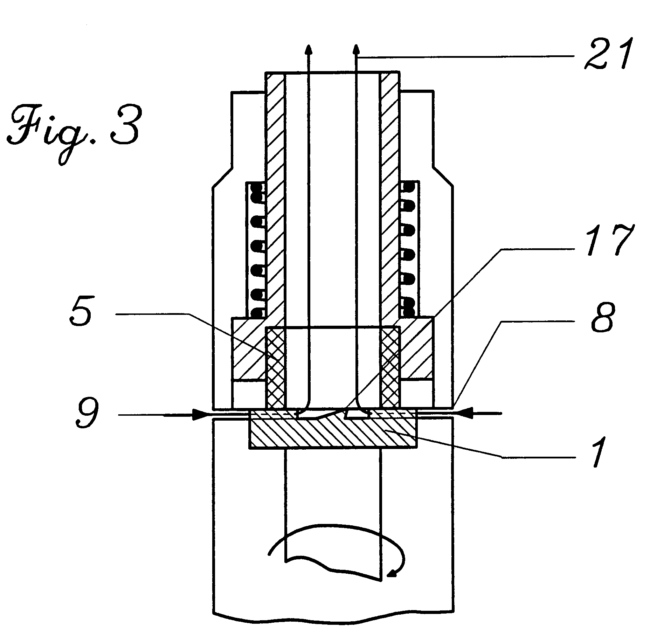

Aus Fig. 3 ist der Strömungspfad der angesaugten Luft und der Wirkstoffpartikel ersichtlich. Durch den ringförmigen Lufteinlaß 8 gelangt die Luft in die Vertiefungen 9 vor und hinter den Schneiden 17 der Stirnfräse 1 und von dort direkt in den durch Wirkstoff-Ringkörper 5 und Inhalationsrohr 2 gebildeten zentrischen Kanal zum Mund des Patienten und in dessen Atemtrakt. Da im Bereich der Stirnfläche des Wirkstoff-Ringkörpers 5 und der Stirnfräse 1 nur ein sehr geringer Querschnitt im Strömungspfad vorhanden ist, entsteht auch bei sehr geringem Saugvolumen des Patienten noch eine für die Inhalation der generierten Wirkstoffpartikel ausreichende Luftgeschwindigkeit.

Das Betätigen der Vorrichtung erfolgt auf folgende Weise:

Mit dem Aufzugsknopf 15 wird die Antriebsfeder 4 gespannt. Gleichzeitig mit dem Einatmen wird durch Drücken des Auslösemechanismusses 12 die Antriebsfeder 4 entriegelt und dadurch die Stirnfräse 1 in Drehung versetzt. Der Drehvorgang dauert nur Bruchteile einer Sekunde.

## Patentansprüche

1. Vorrichtung zur mechanischen Erzeugung inhalierbarer Wirkstoffpartikel durch Abtragen eines ringförmig ausgebildeten und verfestigten Arzneistoffvorrates (5) mit Hilfe eines drehbaren Dosierungsmittels mit einem Luftkanal, der aus dem Bereich des Dosierungsmittels zu einem Mundstück (3) führt welches das Ende eines Inhalationsrohres (2) bildet, dadurch gekennzeichnet, daß als Dosierungsmittel eine antreibbare Stirnfräse (1) dient, gegen die die Stirnseite des ringförmigen Arzneistoffvorrates (5) andrückbar ist und daß der ringförmige Arzneistoffvorrat (5) selbst und die Stirnfräse (1) den Luftweg zum Mundstück (3) bilden daß der Ringkörper (5) und die Stirnfräse (1) von einem Gehäuse umgeben sind und daß das Gehäuse (3, 18) im Bereich der Schneiden (17) der Stirnfräse (1) mit Lufteintrittsöffnungen (8) versehen ist, wobei die Lufteintrittsöffnungen (8), die Vertiefungen (9) zwischen den Schneiden (17) der Stirnfräse (1) und das Inhalationsrohr (2) den zum Mundstück (3) führenden Luftkanal bilden.

2. Vorrichtung nach Anspruch 1, dadurch gekennzeichnet, daß das Inhalationsrohr (2) mit dem Ringkörper (5) mittels einer definierten Andruckfeder (6) auf die Stirnfräse (1) gedrückt wird.

3. Vorrichtung nach Anspruch 2, dadurch gekennzeichnet, daß der Hubweg des Inhalationsrohres (2) mit dem Arzneistoffvorrat (5) begrenzbar ist.

4. Vorrichtung nach Anspruch 2 oder 3, dadurch gekennzeichnet, daß der Ringkörper (5) zusammen mit dem Inhalationsrohr (2) austauschbar angeordnet ist.

5. Vorrichtung nach einem der Ansprüche 2 bis 4, dadurch gekennzeichnet, daß das Inhalationsrohr (2) eine schlüsselartige Kennung aufweist, die jeweils auf ein bestimmtes Arzneimittel abgestimmt ist.

6. Vorrichtung nach einem der Ansprüche 2 bis 5, dadurch gekennzeichnet, daß die Rotation der Stirnfräse (1) durch einen Federantrieb erfolgt.

7. Vorrichtung nach Anspruch 6, dadurch gekennzeichnet, daß die Anzahl der Umdrehungen der Stirnfräse (1) vorbestimmbar ist.

8. Vorrichtung nach Anspruch 6 oder 7, dadurch gekennzeichnet, daß die Vertiefungen (9) vor und hinter den Schneiden (17) der Stirnfräse (1) den kleinsten Querschnitt im gesamten Strömungspfad (21) aufweisen.

## Claims

1. Device for mechanically generating inhalable active substance particles by milling off material from a ring-shaped and compacted drug reservoir (5) by means of a rotatable dosing device with an air duct that leads from the area of the dosing device to a mouth piece (3) that forms the final part of an inhalation tube (2); the device shows the following special characteristics: the dosing device is a face mill (1) with drive, against which the front surface of the ring-shaped drug reservoir (5) can be pressed, and the ring-shaped drug reservoir (5) itself and the face mill (1) form the air duct to the mouth piece (3), and the ring body (5) and the face mill (1) are surrounded by a casing, and the casing (3, 18) is provided with air inlet openings (8) in the area of the cutting edges (17) of the face mill (1), whereby the air inlet openings (8), the identations (9) between the cutting edges (17) of the face mill (1) and the inhalation tube (2) form the air duct leading to the mouth piece (3).

2. Device as per claim 1 with the special characteristic that the inhalation tube (2) together with the ring body (5) is pressed on the face mill (1) by means of a defined contact pressure spring (6).

3. Device as per claim 2 with the special characteristic that the stroke of the inhalation tube (2) with the drug reservoir (5) can be limited.

4. Device as per the claims 2 and 3 with the special characteristic that the ring body (5) is arranged in such a way that it can be replaced together with the inhalation tube (2).

5. Device as per one of the claims 2 to 4 with the special characteristic that the inhalation tube (2) is equipped with a key-type identification that is adjusted to a certain drug respectively.

6. Device as per one of the claims 2 to 5 with the special characteristic that the rotation movement of the face mill (1) is realized by means of a spring drive.

7. Device as per claim 6 with the special characteristic that the number of rotations of the face mill (1) can be predetermined.

8. Device as per claim 6 or 7 with the special characteristic that the indentations (9) before and behind the cutting edges (17) of the face mill (1) have the closest cross section of the entire flow path (21).

## Revendications

1. Dispositif pour générer mécaniquement des particules de matière active, pour l'inhalation en les enlevant d'une réserve de médicament (5), à l'état solide, de forme annulaire, à l'aide d'un agent de dosage tournant, avec un canal d'air qui relie la zone de l'agent de dosage à un embout (3) constituant l'extrémité d'un tube d'inhalation (2),
caractérisé en ce que
• le moyen de dosage est une fraise frontale (1), entraînée, qui peut être pressée contre la face frontale de la réserve de médicament (5), annulaire, et cette réserve de médicament (5) elle-même et la fraise frontale (2) forment le chemin de passage d'air vers l'embout (3),
• le corps annulaire (5) et la fraise frontale (2) sont entourés d'un boîtier,
• le boîtier (3, 18) a des orifices d'entrée d'air (8) dans la zone des arêtes (17) de la fraise frontale (1),
• ces orifices d'entrée d'air (8), les cavités (9) entre les arêtes (17) de la fraise frontale (1) et le tube d'inhalation (2) constituent le canal d'air relié à l'embout (3).

2. Dispositif selon la revendication 1,
caractérisé en ce que
le tube d'inhalation (2) est poussé par un corps annulaire (5) avec un ressort de compression déterminé (6) contre la fraise frontale (1).

3. Dispositif selon la revendication 2,
caractérisé en ce que
la course du tube d'inhalation (2) est limitée par la réserve de médicaments (5).

4. Dispositif selon la revendication 2 ou 3,
caractérisé en ce que
le corps annulaire (5) et le tube d'inhalation (2) sont remplaçables.

5. Dispositif selon l'une des revendications 2 à 4,
caractérisé en ce que
le tube d'inhalation (2) comporte une caractéristique en forme de clé adaptée à un certain type de médicaments.

6. Dispositif selon l'une des revendications 2 à 5,
caractérisé en ce que
la rotation de la fraise frontale (1) est commandée par un entraînement à ressort.

7. Dispositif selon la revendication 6,
caractérisé en ce que
le nombre de rotations de la fraise frontale (1) peut être prédéfini.

8. Dispositif selon les revendications 6 ou 7,
caractérisé en ce que
les cavités (9) en amont et en aval des arêtes (17) de la fraise frontale (1) ont la plus petite section dans l'ensemble du chemin d'écoulement (21).
